# EUROPEAN PATENT APPLICATION

(11) **EP 3 443 939 A1**
(43) Date of publication of application: **20.02.2019**
(21) Application number: 18189689.5
(22) Date of filing: 20.08.2018
(51) Int. Cl.: A61F 2/52

(54) **ARTICLE PROVIDING VISUAL REPLACEMENT NIPPLE - AREOLA COMPLEX**

(30) Priority: 18.08.2017 US 201762547381 P
(71) Applicant: Weir, Stacie-Rae Anna, Calgary, Alberta T2J5J4 (CA)
(72) Inventor: Weir, Stacie-Rae Anna, Calgary, Alberta T2J5J4 (CA)
(74) Representative: Gulde & Partner

(57) **Abstract**

An anatomically accurate device of a human areola and nipple is provided for in the present invention.

## Description

### BACKGROUND OF THE INVENTION

Persons that experience body altering events are typically provided significant amounts of medical and physical care. However, many times emotional care is either not addressed or not fully addressed. A person who has a breast removed either due to cancer or any other reason is often left with a breast region that does not resemble their original body. For many, plastic surgery is not an option.

When it comes to any version of a human-created nipple/Areola complex, a lack of realism can promote negative body dysmorphia.

Negative body dysmorphia is a very real but underestimated problem in breast cancer. Realism is essential to allow the wearer to experience the feelings of completeness and wholeness that she enjoyed prior to the breast amputation.

Realistic likeness of this complex promotes body-positivity by encouraging a deeper level of the self-acceptance process that is crucial to thorough psychological healing.

It is well known that emotional healing is at least as important as physical healing for good quality of life. A realistic visual reminder of wholeness assists a survivor in benefitting from complete healing.

The true personal value of these facts deserves to be recognized.

There is a need for a simple solution to address the missing nipple/areola complex.

### SUMMARY OF THE INVENTION

The present invention, in one embodiment, resembles a standard temporary tattoo with easy application. However, the invention is novel in that there is the beauty of realism in this matter is the emotional impact we can achieve with this advanced attention to detail.

Based from Original, portrait-style drawings of the nipple/Areola complex, there is realism aids in emotional healing by allowing a person to experience an accurate representation of a complete body prior to mastectomy trauma.

Although the disclosure refers to mastectomy, this invention is intended to be used by any person, man or woman that experiences a physical event that alters and/or eliminates the nipple/areola complex.

In a preferred embodiment, the present invention will be constructed and arranged with either materials currently on the US Food and Drug Administration (FDA) list of materials Generally Recognized as Safe (GRAS) or FDA other approved materials including but not limited to materials that are safe to use on irradiated skin after, and possibly during, chemotherapy.

This allows a woman to find that connection very early on in her healing process. A conventional tattoo requires more time for scars to mature making the client wait longer for the results that a temporary tattoo can achieve almost immediately.

In one embodiment, the present invention is an article for use in a person who has an altered or removed nipple / areola complex, said article comprising:
- a base material having a first side and a second side;
- disposing indicia of anatomically accurate topographical features of a nipple / areola complex on said first side; and
- an adhesive on said second side.

In one embodiment, the topographical features are substantially two dimensional.

In one embodiment, the topographical features are substantially three dimensional.

In one embodiment, the adhesive is a removable adhesive.

In one embodiment, the substantially two dimensional indicia is formed to provide visual perception of being three-dimensional.

Additionally, two dimensional indicia is formed to provide visual perception of being three-dimensional is formed using an actual image of the intended user. That is to say, either before an event altering the breast, such as surgery, or after the altering event by taking an image of the noon-altered breast, as in cases where surgery occurs only on one breast.

In one embodiment, the present invention is an article for use in a person who has an altered or removed nipple / areola complex, said article consisting of a base material having a first side and a second side;
disposing indicia of anatomically accurate topographical features of a nipple / areola complex on said first side, wherein the indicia is two dimensional indicia is formed to provide visual perception of being three-dimensional and said indicia is disposed utilizing specific imagery to a specific intended user of said article; and
an adhesive on said second side.

Also contemplated is a method of providing visual replacement of a human nipple areola, said method comprising the steps of:
providing an article according to the disclosure herein;
positioning said adhesive in a desired location on a user's breast; and
applying pressure from the first side in the direction of the users breast, said pressure securing set article into position.

In one embodiment, the present invention is a method including the initial steps of:
obtaining an image of a specific users breast;
using said obtained image to form said anatomically accurate topographical features; and
disposing said indicia being anatomically accurate topographical features form from said image on to said first side of said provided article, followed by;
providing said article with said disposed indicia according to the disclosure herein;
positioning said adhesive in a desired location on a user's breast; and
applying pressure from the first side in the direction of the users breast, said pressure securing set article into position.

### BRIEF DESCRIPTION OF THE SEVERAL VIEWS OF THE DRAWINGS

Figure 1 is a top view of the article according to one embodiment of the present invention.
Figure 2 is a top view of the article according to one embodiment of the present invention.
Figure 3 is a side cross-section view from figure 1.
Figure 4 is a side cross-section view from figure 2.

### DETAILED DESCRIPTION OF THE PREFERRED EMBODIMENT

As provided in the annotations of the accompanying figures, the present invention provides an anatomically accurate structure of human nipple areola complex.

In anatomy, the areola is a small circular area on the body with a different histology from the surrounding tissue. The term, as used herein, denotes the pigmented area on the human breast around the nipple (areola mammae).

Mature human female nipples will reveal several small openings arranged radially around the tip of the nipple (lactiferous ducts) from which milk is released during lactation. Other small openings in the areola are sebaceous glands, known as Montgomery's glands.

The size and shape of areolae and nipples are also highly variable, with those of women usually being larger than those of men and prepubescent girls. Human areolae are mostly circular in shape, but many women and some men have areolae that are noticeably elliptical.

The average diameter of male areolae is around 28.0 mm (1.1 in). Sexually mature women have an average of 38.1 mm (1.5 in), but sizes range up to 100 mm (4 in) or greater.

At the intersection of the areola and the rising edge of the nipple is a fold called the sulcus. It may be a smooth curve of skin, or it may look like a wrinkle.

A demonstrated in the figures, representation of illusory and real characteristics of 3 dimensional nipple/Areola complex including length, width, and depth.

To mimic depth, the present invention includes realism techniques such as creases and folds to illustrate the "nipple erection mechanism" that folds extra skin in the unerected nipple to accommodate the increase in surface area.

Various bumps and dots to exemplify Montgomery/Areola glands, which occur naturally in a real nipple/Areola complex.

In one embodiment, the present invention further includes the method comprising or consisting of the steps of:
initially measuring the nipple / areola complex either prior to removal or from a nipple areola that is not being removed and recording all physically measurable features;
forming an article of the present invention with dimensions congruous to or substantially congruous to the dimensions from said initially measuring;
applying the article on a person at a region where the nipple / areola complex has been altered or removed.

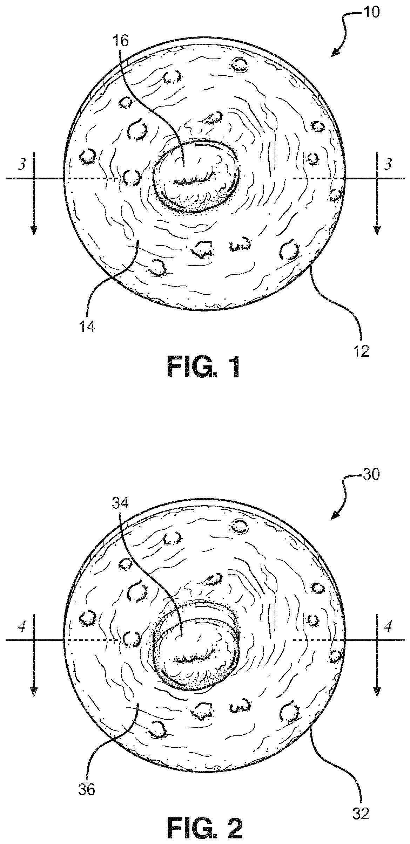

As demonstrated in figure 1 article 10 has a center region whereby depiction of nipple 16 surrounded by areola and indicia 14. As discussed above in one embodiment, the topographical features of the nipple areola complex are disposed on surface 12.

Figure 2 is a side cross-section along sectional lines 3 - 3 from figure 1 and demonstrates the substantially two-dimensional surface of surface 14 and adhesive layer 18 used to secure article 10 into position as desired.

As is known in the visual arts, two dimensional artwork is often provided with significant precision to provide the illusion of a three dimensional figure. In one embodiment of the present invention, surface 14 includes printed indicia that visually appears three-dimensional. in this embodiment, article 10 is not merely a sticker in which a person will place over their own breast, but will provide the visual illusion of a three-dimensional nipple areola complex.

In another embodiment, as discussed above, images of the specific anatomical and topographical features of an individual's breast are used to form the visual indicia on surface 14.

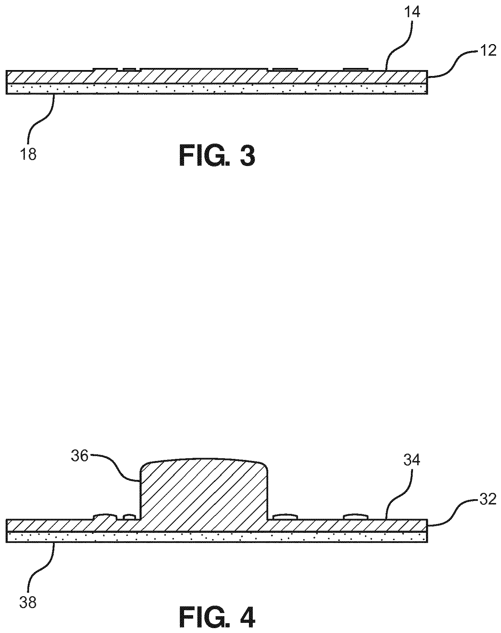

In one embodiment, as demonstrated in figures 2 and 4, the article is formed with actual three-dimensional features on surface 32 with nipple 24 and nipple areola 36.

Figure 4 is a side cross section from figure 2 along sectional lines 4-4.

While the invention has been described in its preferred form or embodiment with some degree of particularity, it is understood that this description has been given only by way of example and that numerous changes in the details of construction, fabrication, and use, including the combination and arrangement of parts, may be made without departing from the spirit and scope of the invention.

## Claims

1. An article for use in a person who has an altered or removed nipple / areola complex, said article comprising;
a base material having a first side and a second side;
disposing indicia of anatomically accurate topographical features of a nipple / areola complex on said first side; and
an adhesive on said second side.

2. The article of claim 1 wherein said topographical features are substantially two dimensional.

3. The article of claim 1 wherein said topographical features are substantially three dimensional.

4. The article of claim 1 wherein said adhesive is a removable adhesive.

5. The article of claim 2 wherein said substantially two dimensional indicia is formed to provide visual perception of being three-dimensional.

6. A method of providing visual replacement of a human nipple areola, said method comprising the steps of:
providing an article according to claim 1;
positioning said adhesive in a desired location on a user's breast; and
applying pressure from the first side in the direction of the users breast, said pressure securing set article into position.

7. The method of claim 6 including an initial steps of:
obtaining an image of a specific users breast;
using said obtained image to form said anatomically accurate topographical features; and
disposing said anatomically accurate topographical features form from said image on to said first side of said provided article.
